# EUROPÄISCHE PATENTSCHRIFT

(11) **EP 3 620 190 B1**
(45) Veröffentlichungstag und Bekanntmachung des Hinweises auf die Patenterteilung: **07.06.2023**
(21) Anmeldenummer: 18192552.0
(22) Anmeldetag: 04.09.2018
(51) Int. Cl.: A61M 1/14, A61M 1/36

(54) **HÄMODIALYSE-GERÄTEANORDNUNG**
HEMODIALYSIS DEVICE ASSEMBLY
ENSEMBLE D'APPAREILS D'HÉMODIALYSE

(43) Veröffentlichungstag der Anmeldung: 11.03.2020
(73) Patentinhaber: Nipro Corporation, Osaka 531-8510 (JP); Nipro Medical Europe N.V., 2800 Mechelen (BE)
(72) Erfinder: Breuch, Gerd, 20253 Hamburg (DE); Breuch, Julius, 22765 Hamburg (DE); Holzschuh, Robert, 22081 Hamburg (DE); Lange, Christian, 20359 Hamburg (DE); Biermann, Frank, 22455 Hamburg (DE)
(74) Vertreter: terpatent Patentanwälte ter Smitten Eberlein-Van Hoof Rütten Daubert

(56) Entgegenhaltungen:
- DE-A1-102010 032 179
- US-A1- 2017 021 086

## Beschreibung

Die Erfindung bezieht sich auf eine Hämodialyse-Geräteanordnung mit einem Basisgerät, das ein Gerätegehäuse und eine Internfluidik innerhalb des Gerätegehäuses aufweist, und mit einem eine Externfluidik bildenden Externfluidik-Set außerhalb des Gerätegehäuses, das über kuppelbare Fluidkupplungen fluidisch mit dem Basisgerät bzw. der Internfluidik verbunden ist.

Eine Hämodialyse-Geräteanordnung nach dem Stand der Technik, wie sie beispielsweise aus DE 10 2010 032 179 A1 bekannt ist, ist in der Regel fluidisch zweigeteilt in eine Externfluidik und eine Internfluidik. Die Internfluidik ist für den vielfachen und dauerhaften Gebrauch ausgelegt und besteht daher aus standfesten Materialien, die desinfizierbar sind. Die Internfluidik weist in aller Regel keine blutführenden Leitungen auf. Die Externfluidik dagegen weist unter anderem alle blutführenden Leitungen und Komponenten des sogenannten extrakorporalen Blutkreislaufs auf. Die Externfluidik ist schon zur Vermeidung von blutgestützten Infektionen ausschließlich für den Einmalgebrauch ausgelegt.

Die Internfluidik ist zum größten Teil oder vollständig innerhalb des Gerätegehäuses angeordnet, das in der Regel werkzeuglos nicht geöffnet werden kann. Die Externfluidik dagegen ist in der Regel auf einem Bediener-Interface angeordnet, das frei zugänglich oder werkzeuglos zugänglich ist. Das Interface weist beispielsweise häufig eine mechanische Schlauchpumpen-Mimik auf, in die ein Schlauch der Externfluidik eingelegt werden kann, um auf diese Weise eine Pumpe zu bilden, beispielsweise die Blutpumpe im extrakorporalen Blutkreis der Externfluidik.

Die fluidische Verbindung zwischen der Internfluidik und der Externfluidik wird in der Regel über fluidische Kupplungen hergestellt, die vereinzelt ausgebildet sind.

Der komplette Arbeitsablauf einer Dialysebehandlung eines Patienten beginnt mit dem Applizieren der als Leitungsset ausgebildeten Externfluidik an dem Basisgerät bzw. dem Interface, um die Externfluidik und insbesondere den extrakorporalen Blutkreis zu desinfizieren und vorzufüllen mit einer Vorfüllflüssigkeit. Danach muss die Externfluidik umgesteckt werden, um das venöse und das arterielle Leitungsende mit dem arteriellen und dem venösen Patientenzugang fluidisch zu verbinden, um die Blutreinigung starten zu können. Nach dem Abschluss der Blutreinigung wird die Externfluidik erneut umgesteckt, um das Blut aus dem extrakorporalen Blutkreis der Externfluidik vollständig zurück zum Patienten zu pumpen.

Während des kompletten Behandlungszyklus' findet also ein mehrfaches Umstecken der Leitungen der Externfluidik statt, was u.a. über entsprechende Fluidkupplungen an dem Interface erfolgt. Grundsätzlich besteht hierbei die Gefahr von falschen Verkupplungen. Ferner ist das Umstecken von vielen Einzel-Fluidkupplungen relativ zeitaufwendig.

Aus US 2007/0021086 A1 ist ein Hämodialyse-Basisgerät bekannt, bei dem die Flüssigkeits-Container über Koaxial-Kupplungen mit der Internfluidik des Basisgeräts verbunden sind, um auf diese Weise für einen Druckausgleich innerhalb der Container zu sorgen.

Aufgabe der Erfindung ist es demgegenüber, die Handhabung der Externfluidik einer Hämodialyse-Geräteanordnung während des gesamten Behandlungszyklus' zu vereinfachen.

Diese Aufgabe wird erfindungsgemäß gelöst mit einer Hämodialyse-Geräteanordnung mit den Merkmalen des Anspruchs 1.

Die Hämodialyse-Geräteanordnung besteht aus einem Basisgerät, das ein Gerätegehäuse und eine stationäre Internfluidik innerhalb des Gerätegehäuses aufweist, und einem eine nicht-stationäre Externfluidik bildenden Externfluidik-Set außerhalb des Gerätegehäuses. Die Externfluidik ist vorzugsweise vollständig aus Kunststoff-Teilen bestehend und ist über kuppelbare Fluidkupplungen fluidisch mit der Internfluidik des Basisgeräts verbunden. Der Begriff "stationär" bezieht sich vorliegend stets auf das Basisgerät, das seinerseits selbstverständlich beweglich ausgebildet sein kann. Die Fluidkupplungen sind bevorzugt an einem Interface des Basisgeräts angeordnet, beispielsweise an einer frei zugänglichen Gerätefront. Die Externfluidik weist die den extrakorporalen Blutkreislauf bildenden Leitungen auf und kann als Einmal-Artikel ausgebildet sein, kann aber auch mehrmals verwendbar und desinfizierbar ausgebildet sein. Unter einem Fluid ist vorliegend eine Flüssigkeit, ein Gas oder ein Gemisch hieraus zu verstehen.

Gemäß der Erfindung ist eine fluidische Koaxial-Doppelport-Kupplung vorgesehen, die von einer Basisgerät-seitigen stationären Doppelport-Buchse und einem korrespondierenden Externset-seitigen Doppelport-Stecker gebildet ist, der auf die Doppelport-Buchse lösbar aufgesteckt ist. Der Doppelport-Stecker und die Doppelport-Buchse weisen jeweils eine fluidische Zentralleitung und eine die Zentralleitung jeweils ringförmig umgebende koaxiale fluidische Ringleitung auf. Im zusammengekuppelten Zustand der Kupplung sind die beiden Zentralleitungen und die beiden Ringleitungen jeweils fluidisch miteinander verbunden. Unter "ringförmig" ist vorliegend nicht zwangsweise ein Kreisring zu verstehen, sondern kann auch ein Oval, ein Rechteck etc. verstanden werden.

Die Begriffe "Buchse" und "Stecker" haben vorliegend keine besondere Bedeutung im Hinblick darauf, welches Teil in welches Teil eingesteckt wird, sondern die beiden voneinander abweichenden Begriffe werden lediglich zur einfacheren Beschreibung und Unterscheidung verwendet. Mit dem Begriff "Stecker" wird zum Ausdruck gebracht, dass dies der nicht-stationäre, also mobile Teil der Kupplung ist, der aktiv an die stationäre Buchse gesteckt bzw. von dieser gelöst und entfernt wird.

Unter einer Kupplung ist vorliegend eine Kupplungs-Mimik zu verstehen, die durch einfache Handgriffe eine sichere mechanische und fluidische Verbindung sicherstellt oder wieder lösbar ist, beispielsweise in Form einer Bajonettkupplung oder einer Überwurfmutter-Kupplung.

Durch die Einrichtung einer koaxialen Doppelport-Kupplung werden zwei unverwechselbare einzelne fluidische Kupplungen zusammengefasst und eindeutig definiert, so dass eine eindeutige fluidische Zuordnung der beiden Fluidanschlüsse definiert ist und eine Verwechslung ausgeschlossen werden kann. Hierdurch wird die Bedienungssicherheit erheblich verbessert. Ferner werden mit einem einzigen (Ent-) Kupplungsvorgang zwei fluidische Verbindungen hergestellt bzw. voneinander getrennt, was die Handhabung während des gesamten Arbeitsablaufs der Dialysebehandlung vereinfacht und beschleunigt.

Dem Basisgerät ist fluidisch eine Substituatflüssigkeits-Quelle zugeordnet, die fluidisch mit der Buchsen-Ringleitung oder der Buchsen-Zentralleitung verbunden ist. Die Substituatpumpe kann grundsätzlich als externe Pumpe ausgebildet sein. Besonders bevorzugt weist das Basisgerät eine interne Substituatpumpe auf, die die Substituatflüssigkeit von der Substituatflüssigkeits-Quelle zu der Doppelport-Kupplung pumpt. Über die Basisgerät-seitige Doppelport-Buchse kann also Substituatflüssigkeit von der Substituatflüssigkeits-Quelle in den extrakorporalen Blutkreis des Externfluidik-Sets eingespeist werden. Die Substituatflüssigkeit kann jedoch zusätzlich auch zum Vorfüllen des extrakorporalen Blutkreises der Externfluidik genutzt werden. Schließlich kann die Substituatflüssigkeit auch nach der eigentlichen Blutbehandlung zum Ausschieben der Blutsäule aus dem extrakorporalen Blutkreis der Externfluidik zurück zum Patienten genutzt werden.

Dem Basisgerät ist ein Abfallflüssigkeits-Ziel zugeordnet, das fluidisch mit der Buchsen-Zentralleitung oder der Buchsen-Ringleitung verbunden ist.

Auf diese Weise kann die Koaxial-Doppelport-Kupplung beispielsweise zur Herstellung der fluidischen Verbindungen zwischen der Externfluidik und der Internfluidik genutzt werden, die für das Vorfüllen des extrakorporalen Kreises der Externfluidik oder für das Spülen oder Desinfizieren des extrakorporalen Blutkreises der Externfluidik erforderlich sind. Besonders vorteilhaft ist es, die Doppelport-Buchse auf der Geräteseite mit der Substituatflüssigkeits-Quelle einerseits und dem Abfallflüssigkeits-Ziel andererseits fluidisch zu verbinden. Unter dem Abfallflüssigkeits-Ziel kann vorliegend beispielsweise das öffentliche Abwassernetz oder ein interner Abwasserbehälter verstanden werden.

Gemäß einer bevorzugten Ausgestaltung der Erfindung ist geräteseitig ein Verriegelungsmittel vorgesehen, wobei das Verriegelungsmittel in seiner Verriegelungsposition den Doppelport-Stecker in seiner Kupplungsposition auf der stationären Doppelport-Buchse blockiert. Durch das Verriegelungsmittel ist auf einfache Weise für den Nutzer sichtbar, dass die Doppelport-Kupplung verkuppelt und verriegelt ist. Ferner wird durch das geräteseitige Verriegelungsmittel die Möglichkeit geschaffen, die korrekte Verriegelung der Doppelport-Kupplung zu kontrollieren. Das Verriegelungsmittel kann beispielsweise als Schwenkbügel ausgebildet sein, der über den angekuppelten Doppelport-Stecker geschwenkt werden kann, um den Doppelport-Stecker in seiner Verriegelungsposition axial und/oder rotatorisch zu verriegeln. Da das Verriegelungsmittel fester Bestandteil des Basisgeräts und kein Einmalartikel ist, können die Herstellungs-Kosten für die Einmalartikel-Externfluidik auf diese Weise gering gehalten werden. Der Doppelport-Stecker und die korrespondierende Kupplung können mit einer Luer-Mimik ausgestaltet sein.

Besonders bevorzugt weist das Verriegelungsmittel eine Flussumkehr-Kappe auf, die bei nicht angekoppeltem Doppelport-Stecker in einer Spülposition des Verriegelungsmittels die Doppelport-Buchse derart verschließt, dass die Buchsen-Zentralleitung fluidisch verbunden ist mit der Buchsen-Ringleitung. Alternativ kann die Flussumkehr-Kappe jedoch auch getrennt von dem Verriegelungsmittel ausgebildet sein. Mit der geräteseitig vorgesehenen Flussumkehr-Kappe wird es ermöglicht, die Buchsen-Zentralleitung und die Buchsen-Ringleitung sowie die mechanische Buchsenkupplung gleichzeitig zu spülen bzw. zu desinfizieren. Ferner wird bei Nichtgebrauch der Doppelport-Kupplung diese durch die Flussumkehr-Kappe vor Kontamination und mechanisch geschützt.

Vorzugsweise ist dem Verriegelungsmittel und/oder der Flussumkehr-Kappe ein Positionsdetektor zugeordnet, der die Verriegelungsposition und/oder die Spülposition des Verriegelungsmittels detektiert. Auf diese Weise wird eine hohe Funktionssicherheit und Prozesssicherheit hergestellt.

Vorzugsweise weist das nicht-stationäre Externset einen Doppelport-Adapter mit dem Doppelport-Stecker und eine separate Blutkreis-Leitungsanordnung auf, die während der Hämodialyse-Behandlung den extrakorporalen Blutkreis bildet. Die Blutkreis-Leitungsanordnung weist an ihrem arteriellen und an ihrem venösen Leitungsende eine arterielle Kupplungsbuchse und eine venöse Kupplungsbuchse auf, die an korrespondierende Kupplungsstecker an dem Patienten ankuppelbar sind. Der Doppelport-Adapter weist einen korrespondierenden arteriellen Kupplungsstecker auf, der fluidisch mit der Substituatflüssigkeits-Quelle verbunden ist und weist einen korrespondierenden venösen Kupplungsstecker auf, der fluidisch mit dem Abfallflüssigkeitsbehälter verbunden ist. Die beiden Kupplungsbuchsen der Blutkreis-Leitungsanordnung können zum Vorfüllen der Blutkreis-Leitungsanordnung an den Doppelport-Adapter fluidisch angekuppelt werden, sodass die Blutkreis-Leitungsanordnung mit einer geeigneten Vorfüllflüssigkeit, beispielsweise mit der Substituatflüssigkeit, vorgefüllt werden kann. Hierbei fließt die geeignete Vorfüllflüssigkeit in das arterielle Leitungsende der Blutkreis-Leitungsanordnung ein und fließt schließlich durch das venöse Leitungsende zu dem geräteseitigen Abfallflüssigkeitsbehälter. Nach dem Vorfüllen der Blutkreis-Leitungsanordnung, durch das die gesamte Luft aus der Blutkreis-Leitungsanordnung entfernt ist, können die beiden Leitungsenden an die korrespondierenden Kupplungen der beiden patientenseitigen Zugänge angekuppelt werden.

Vorzugsweise weist die Blutkreis-Leitungsanordnung einen separaten Substituatzugang zwischen den beiden Blutkreis-Leitungsenden auf, der von einer arteriellen Kupplungsbuchse gebildet wird. Die arterielle Kupplungsbuchse des Substituatzugangs ist also baugleich mit der arteriellen Kupplungsbuchse an dem arteriellen Leitungsende. Auf diese Weise kann die Substituatquelle nach dem Vorfüllen und während der Dialysebehandlung einfach mit dem separaten Substituatzugang der Blutkreis-Leitungsanordnung verkuppelt, also fluidisch verbunden werden. Durch die unterschiedliche Ausbildung der arteriellen Kupplungsbuchse und der venösen Kupplungsbuchse kann eine fehlerhafte Verkupplung praktisch ausgeschlossen werden, wodurch die Bedienungssicherheit erheblich erhöht ist.

Vorzugsweise ist in der Blutkreis-Leitungsanordnung stromaufwärts des venösen Leitungsendes und vorzugsweise stromabwärts des Dialysators ein Rückschlagventil angeordnet, das in Flussrichtung zum venösen Leitungsende öffnet und in entgegengesetzter Flussrichtung schließt. Hierdurch wird bei fehlerhafter Applizierung der Blutkreis-Leitungsanordnung eine Fehlfunktion verhindert.

Im Folgenden wird ein Ausführungsbeispiel der Erfindung anhand der Zeichnungen näher erläutert. Es zeigen:
Figur 1 eine schematische Darstellung einer erfindungsgemäßen Hämodialyse-Geräteanordnung einschließlich einer Koaxial-Doppelport-Kupplung, durch die die Internfluidik des Basisgeräts mit der Externfluidik fluidisch verbunden ist, beim Vorfüllen,
Figur 1a eine vergrößerte Darstellung des Details Ia der Figur 1,
Figur 2 die Hämodialyse-Geräteanordnung der Figur 1 während der Blutreinigung,
Figur 3 die Hämodialyse-Geräteanordnung der Figur 1 während der Blutrückgabe,
Figur 4 die Doppelport-Kupplung der Figur 1 während des Spülens der Basisgerät-seitigen stationären Doppelport-Buchse,
Figur 5 einen ersten Längsschnitt der Doppelport-Kupplung der Hämodialyse-Geräteanordnung der Figur 1,

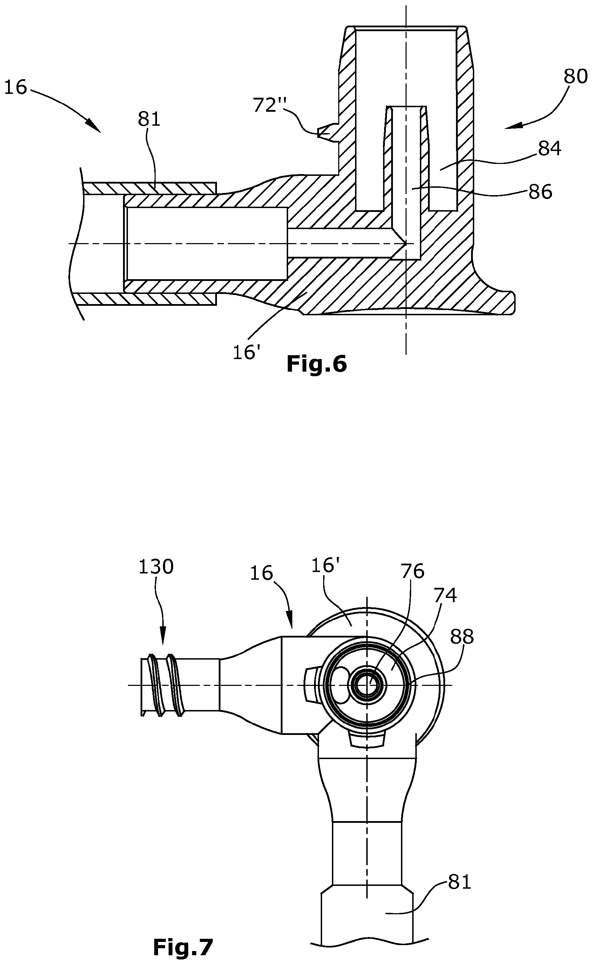

Figur 6 einen zweiten Längsschnitt des Doppelport-Adapters 16 der Doppelport-Kupplung der Figur 5, und
Figur 7 eine Draufsicht auf den Doppelport-Adapter der Figur 6.

In den Figuren ist eine Hämodialyse-Geräteanordnung 10 dargestellt, mit der an einem Patienten eine Blutreinigung vorgenommen werden kann. Die Hämodialyse-Geräteanordnung 10 ist strukturell aufgeteilt in ein stationäres Basisgerät 12, das ein geschlossenes Gerätegehäuse 20 aufweist, in dem eine Internfluidik angeordnet ist. Außerhalb des Gerätegehäuses 20 weist die Hämodialyse-Geräteanordnung 10 eine Externfluidik auf, die von einem Externfluidik-Set 14 gebildet wird, das im Wesentlichen aus einem Doppelport-Adapter 16 und einer Blutkreis-Leitungsanordnung 18 besteht. Das Externfluidik-Set 14 ist nicht wiederverwendbar, sondern ist als Einmal-Artikel ausgebildet, der nur für eine einzige Dialysebehandlung benutzt und danach entsorgt wird. Das Gerätegehäuse 20 ist grundsätzlich geschlossen ausgebildet und kann nicht ohne weiteres werkzeuglos geöffnet werden, sodass für den Benutzer und den Patienten kein unmittelbarer Zugriff auf die Internfluidik besteht.

Das Gerätegehäuse 20 weist an seiner dem Bediener zugewandten Frontseite ein Gerätegehäuse-Interface 22 auf, das die Schnittstelle zwischen der Externfluidik und der Internfluidik darstellt und mehrere Fluid-Kupplungen für die fluidische Verbindung der Internfluidik mit der Externfluidik aufweist.

Die Internfluidik und die Externfluidik sind unter anderem über eine Koaxial-Doppelport-Kupplung 15 fluidisch miteinander verbunden, durch die zwei einzelne fluidische Verbindungen zwischen der Internfluidik und der Externfluidik bedarfsweise hergestellt werden.

Das Basisgerät 12 weist eine Substituatflüssigkeits-Quelle 24 auf, die vorliegend als interner Substituatflüssigkeits-Tank 25' ausgebildet ist, der eine Substituatflüssigkeit 25 enthält. Der Tank 25' kann alternativ auch außerhalb des Basisgerät- Gehäuses 20 angeordnet sein. Stromabwärts der Substituatflüssigkeits-Quelle 24 ist eine Substituatpumpe 31 angeordnet, durch die die Substituatflüssigkeit 25 zu einem Pumpziel gepumpt werden kann. Ferner weist das Basisgerät 12 innerhalb des Gerätegehäuses 20 eine Vorfüllflüssigkeits-Quelle 26 auf, die von einem Tank 27' gefüllt mit einer Vorfüllflüssigkeit 27 gebildet wird. Der Vorfüllflüssigkeits-Tank 27' kann alternativ auch außerhalb des Gerätegehäuses 20 angeordnet sein. Stromabwärts der Vorfüllflüssigkeit-Quelle 26 ist eine Vorfüllflüssigskeitspumpe 32 angeordnet, die die Vorfüllflüssigkeit 27 zu einem Vorfüllflüssigkeits-Ziel pumpen kann. Die Leitungen jeweils stromabwärts der beiden vorgenannten Pumpen 31,32 sind in einem Dreiwege-Quellenwahl-Ventil 36 zusammengeführt, dessen abgehender dritter Zweig zu der Koaxial-Doppelport-Kupplung 15 führt. Ferner weist das Basisgerät 12 einen Abfallflüssigkeitsbehälter 28' als Abfallflüssigkeits-Ziel 28 für Abfallflüssigkeit 29 auf.

Das Externfluidik-Set 14 außerhalb des Gerätegehäuses 20 weist eine den extrakorporalen Blutkreis bildende und während der Blutreinigung blutführende Leitung 62 auf, die zwischen ihrem arteriellen Leitungsende 68 und ihrem venösen Leitungsende 69 von drei Leitungsabschnitten 62', 62", 62‴ gebildet wird. Die blutführende Leitung 62 besteht aus Kunststoff, ist transparent und plastisch verformbar ausgebildet. Im an das Basisgerät 12 bzw. an das Gerätegehäuse-Interface 22 applizierten Zustand des Internfluidik-Sets 14 ist der erste Leitungsabschnitt 62' in die Blutpumpen-Mechanik 50' einer peristaltischen Blutpumpe 50 eingelegt, wobei die Blutpumpen-Mechanik 50' von einem Blutpumpen-Antriebsmotor 52 innerhalb des Gerätegehäuses 20 angetrieben wird. Stromabwärts der Blutpumpen-Mechanik 50 ist ein erster Substituatzugang 63 vorgesehen, der von einer arteriellen Kupplungsbuchse 121 gebildet wird. Stromabwärts des ersten Substituatzugangs 63 mündet die blutführende Leitung 62 in einen Dialysator 60, der eine großflächige Membran aufweist, durch die harnpflichtige Substanzen aus dem Blut auf die andere Seite der Membran in eine Dialyseflüssigkeit diffundieren können, die dem Dialysator 60 von einer Dialyseflüssigkeits-Versorgung 13 innerhalb des Gerätegehäuses 20 zur Verfügung gestellt wird.

Stromabwärts des Dialysators 60 kann in einem zweiten Leitungsabschnitt 62" ein alternativer oder ein zusätzlicher zweiter Substituatzugang 64 vorgesehen sein, der von einer arteriellen Kupplungsbuchse 122 gebildet wird. Stromabwärts des zweiten Substituatzugangs 64 mündet der zweite Leitungsabschnitt 62" in eine Luftfalle 67, die einen dritten Substituatzugang 65 aufweist, der von einer arteriellen Kupplungsbuchse 123 gebildet wird. Stromabwärts der Luftfalle 67 ist im Verlauf des dritten Leitungsabschnitts 62‴ ein Rückschlagventil 66 angeordnet, sodass eine Flüssigkeit in der blutführenden Leitung 62 stets nur in einer Richtung von dem arteriellen Leitungsende 68 zu dem venösen Leitungsende 69 fließen kann. Stromabwärts des Rückschlagventils 66 mündet der dritte Leitungsabschnitt 62ʺʺ in einen venösen Kupplungsstecker 130, der das venöse Leitungsende 69 bildet.

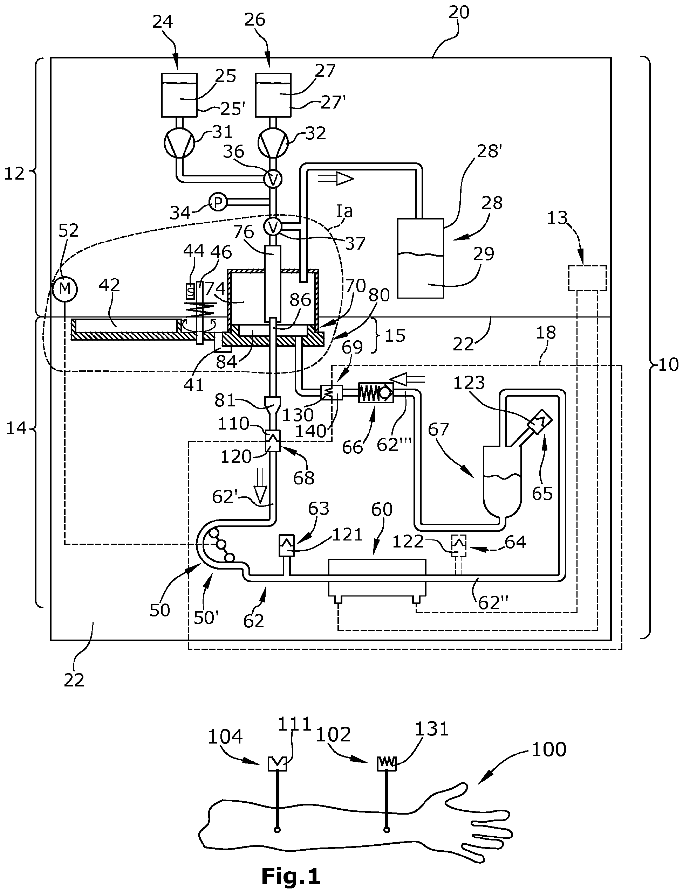

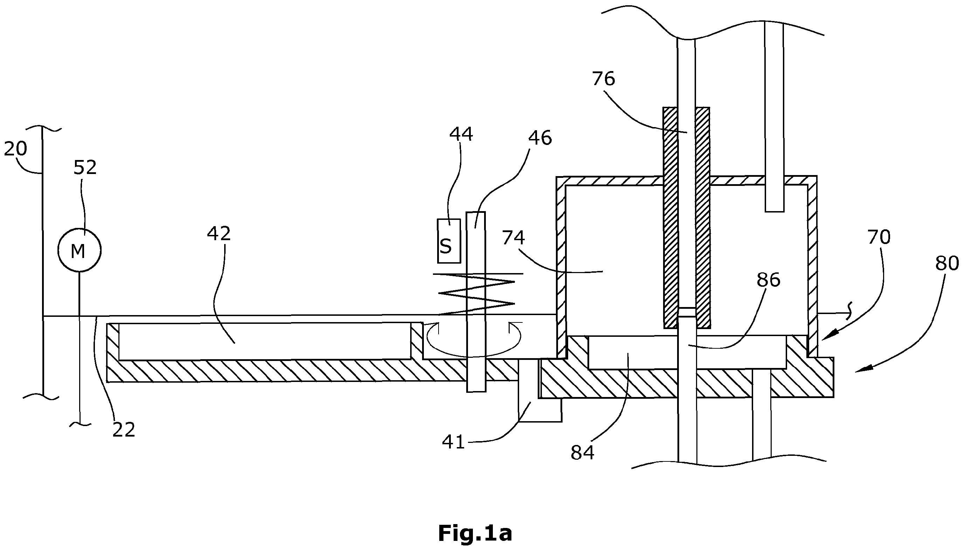

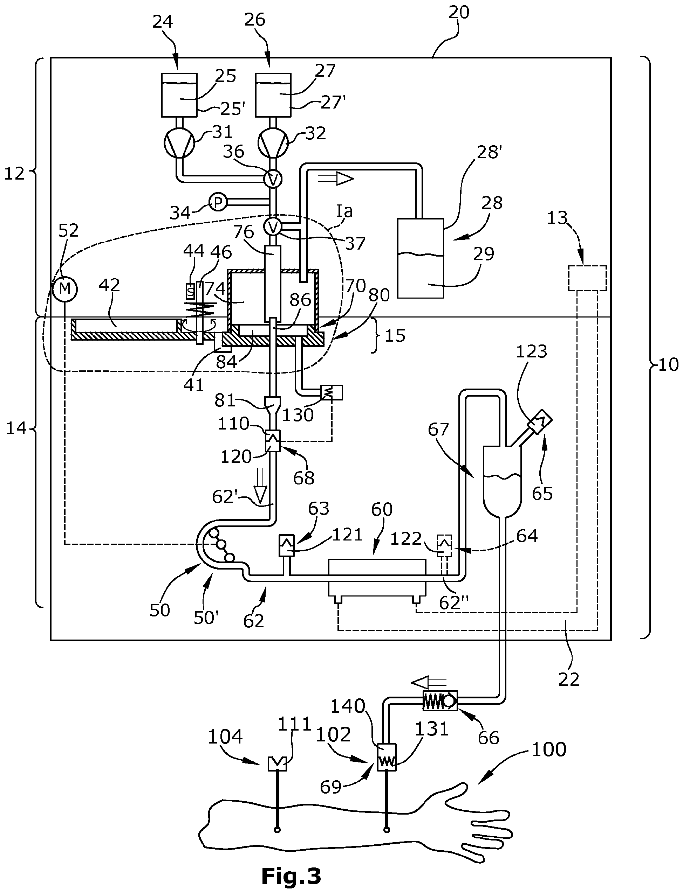

In den Figuren 1-3 ist ferner ein Patient 100 dargestellt, an dessen Arm ein arterieller Patientenzugang 102 mit einem arteriellen Kupplungsstecker 111 und ein venöser Patientenzugang 104 mit einem venösen Kupplungsstecker 131 appliziert ist.

Die Koaxial-Doppelport-Kupplung 15 besteht aus einem internfluidischen Teil und einem externfluidischen Teil. Der internfluidische Teil der Koaxial-Doppelport-Kupplung 15 definiert eine Basisgerät-seitige Doppelport-Buchse 70, die eine fluidische Zentralleitung 76 und eine die Zentralleitung 76 ringförmig umgebende koaxiale Ringleitung 74 aufweist. Der Externfluidik-Teil der Koaxial-Doppelport-Kupplung 15 wird von einem Doppelport-Adapter 16 gebildet, der von einem Kunststoff-AdapterKörper 16' definiert ist, der wiederum eine fluidische Zentralleitung 86 und eine die Zentralleitung 86 ringförmig umgebende koaxiale Ringleitung 84 bildet.

Wenn der Doppelport-Adapter 16 fluidisch appliziert ist, wie in den Figuren 1-3 und 5 dargestellt ist, sind die beiden Ringleitungen 74, 84 und die beiden Zentralleitungen 76, 86 jeweils fluidisch miteinander verbunden, so dass zwei voneinander unabhängige fluidische Verbindungen zwischen der Internfluidik und der Externfluidik hergestellt sind. Die Stecker-Zentralleitung 86 mündet in eine flexible Stecker-Anschlussleitung 81, die an ihrem Leitungsende einen arteriellen Kupplungsstecker 110 aufweist, der mit allen arteriellen Kupplungsbuchsen 120-123 aufgrund seiner mechanischen Kodierung grundsätzlich zusammenkuppelbar ist. Die Stecker-Ringleitung 84 mündet fluidisch in einen venösen Kupplungsstecker 130, der mit der venösen Kupplungsbuchse 140 zusammenkuppelbar ist.

Unmittelbar benachbart zu der Doppelport-Kupplungsbuchse 70 ist geräteseitig an dem Interface 22 ein schwenkhebelartiges Verriegelungsmittel 40 angeordnet. Das Verriegelungsmittel 40 weist eine schwenkbare und axial verschiebbare Verriegelungsmittel-Welle 46 auf, die außenseitig von einem stationären Positionssensor 44 lesbare Markierungen aufweist, so dass der Positionssensor 44 drei verschiedene Positionen des Verriegelungsmittels 40 zuverlässig detektieren kann. Der Verriegelungsmittel-Welle 46 ist eine Vorspannfeder 47 beispielsweise koaxial zugeordnet, sodass die Welle 46 in proximaler Richtung axial vorgespannt ist. Die drei möglichen Positionen des Verriegelungsmittels 40 sind die funktionslose Position, die Verriegelungsposition und die Spülposition, die im Folgenden noch erläutert werden.

An der Verriegelungsmittel-Welle 46 schließt sich ein hebelartiges Gebilde an, das eine deckelartige Flussumkehr-Kappe 42 und ein Verriegelungsteil 41 bildet. In der Verriegelungsposition des Verriegelungsmittels 40 ist der Doppelport-Kupplungsstecker 80 in seiner Verriegelungsposition durch das Verriegelungsteil 41 blockiert, sodass der Doppelport-Kupplungsstecker 80 nicht versehentlich von der Doppelport-Kupplungsbuchse 70 entfernt werden kann.

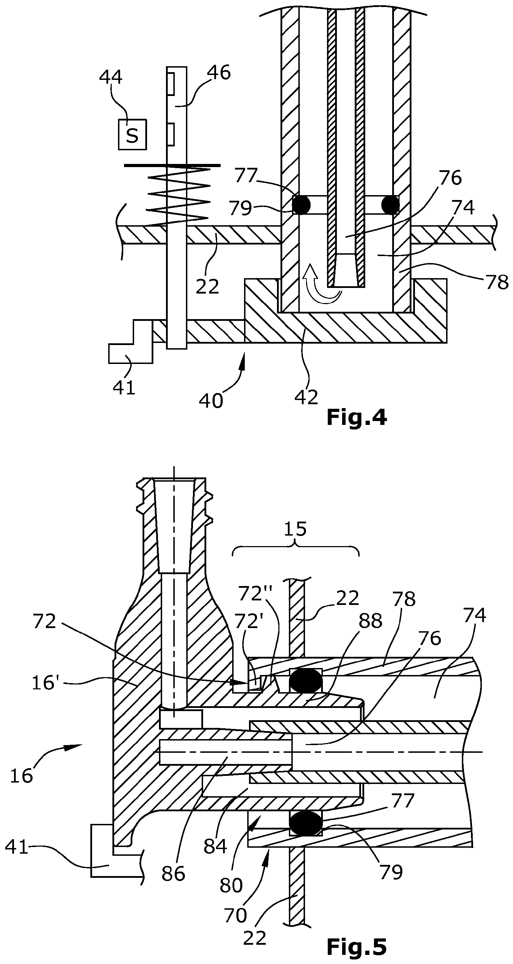

Wie in der Figur 5 dargestellt ist, ist an der Innenseite der äußeren zylindrischen Buchsenwand 78 der Kupplungsbuchse in einer zirkulären Dichtring-Nut 79 ein Dichtring 77 angeordnet, der die zuverlässige fluiddichte Abdichtung mit der korrespondierenden zylindrischen Steckerwand 88 sicherstellt. Im zusammengekuppelten Zustand ist ferner die Stecker-Zentralleitung 86 fluiddicht in die Buchsen-Zentralleitung 76 eingesteckt, wobei die beiden Zentralleitungsenden leicht konisch angeschrägt ausgebildet sind und fluiddicht aneinander anliegen.

Die Doppelport-Kupplungsbuchse 70 und der Doppelport-Kupplungsstecker 80 weisen eine miteinander korrespondierende Bajonettverriegelung 72 auf, die von einem steckerseitigen Bajonettsteg 72", der nach radial außen abragt, und einem buchsenseitigen Bajonettring 72' gebildet wird. Im zusammengekuppelten Zustand hintergreift der Bajonettsteg 72" den Bajonettring 72', wie in Fig. 5 dargestellt.

In der Figur 1 ist die Hämodialyse-Geräteanordnung 10 beim Vorfüllen dargestellt: Der arterielle Kupplungsstecker 110 und der venöse Kupplungsstecker 130 des Doppelport-Adapters 16 sind mit der korrespondierenden arteriellen Kupplungsbuchse 120 des arteriellen Leitungsendes 68 und mit der korrespondierenden venösen Kupplungsbuchse 140 des venösen Leitungsendes 69 verkuppelt und fluidisch verbunden. Die Vorfüllflüssigkeit 27 wird durch die aktivierte Vorfüllflüssigkeitspumpe 32 und die ebenfalls aktivierte Blutpumpe 50 aus einer Richtung in die Blutleitung 62 gepumpt, sodass die Blutleitung 62 schließlich vollständig mit Vorfüllflüssigkeit gefüllt ist. Überschüssige Vorfüllflüssigkeit fließt schließlich in den Abfallflüssigkeitsbehälter 28 ab.

Alternativ kann die Blutleitung 62 beidseitig gefüllt werden. Hierzu wird das Synchronfüllungs-Ventil 37 so umgeschaltet, dass die Vorfüllflüssigkeit sowohl von dem arteriellen Leitungsende 68 als auch von dem venösen Leitungsende 69 aus in die Blutleitung 62 gepumpt wird. Dabei pumpt die Blutpumpe 50 ungefähr mit der halben Flussleistung wie die Vorfüllflüssigkeitspumpe 32, wobei die Luft aus der Blutleitung 62 beispielsweise über den Substituatzugang 65 der Luftfalle 67 entweicht.

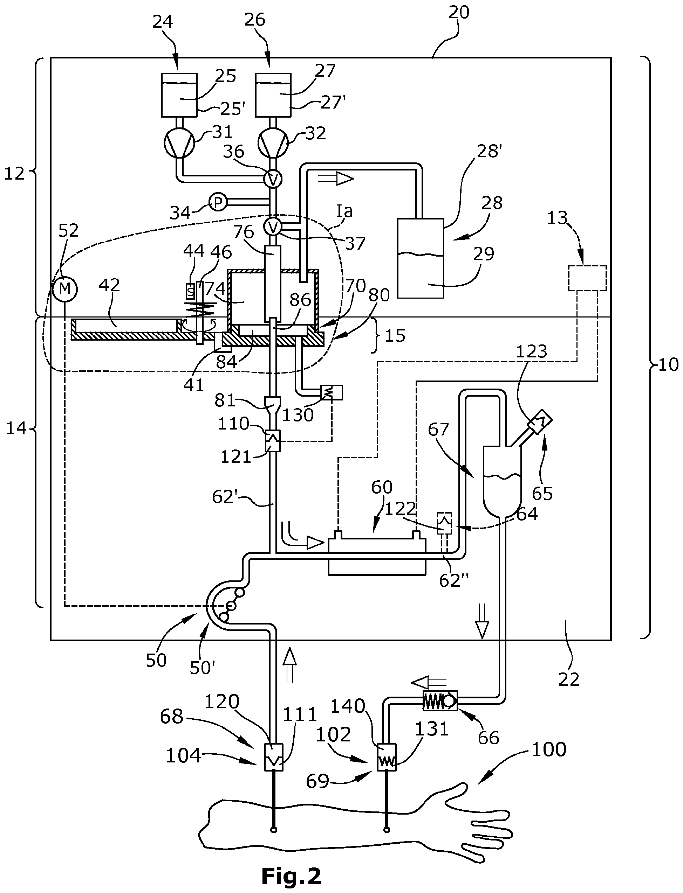

Sobald die Blutleitung 62 vollständig mit Vorfüllflüssigkeit gefüllt ist, werden die beiden Pumpen 32,50 angehalten, und werden die beiden Leitungsenden 68,69 an dem Patienten 100 appliziert, wie in Figur 2 dargestellt. Ferner wird der arterielle Kupplungsstecker 110 der Koaxial-Doppelkupplung 15 an einen Substituatzugang 63 appliziert, so dass bei Bedarf mit aktivierter Substituatpumpe 31 Substituatflüssigkeit 25 aus der Substituatflüssigkeits-Quelle 24 über den Substituatzugang 63 in die Blutleitung 62 eingeleitet werden kann.

Nach Abschluss der eigentlichen Blutreinigung wird die arterielle Kupplungsbuchse 120 des arteriellen Leitungsendes 68 der Blutkreis-Leitungsanordnung 18 wieder an den arteriellen Kupplungsstecker 110 der Koaxial-Doppelkupplung 15 angeschlossen. Die Substituatflüssigkeits-Pumpe 31 wird aktiviert, sodass die Substituatflüssigkeit 25 von dem arteriellen Leitungsende 68 aus in die Blutleitung 62 gepumpt wird, wodurch das Blut aus der Blutleitung 62 über das venöse Leitungsende 69 zurück in den Patienten 100 gepumpt wird.

Nach Abschluss der Dialysebehandlung wird der Doppelport-Adapter 16 entfernt und entsorgt. Anschließend wird das Verriegelungsmittel 40 so verschwenkt, dass die Flussumkehr-Kappe 42 die Öffnung der Doppelport-Kupplungsbuchse 70 vollständig verschließt. Anschließend kann eine Spülung und/oder eine Desinfektion der Doppelport-Kupplungsbuchse 70 erfolgen, indem eine Spülflüssigkeit, beispielsweise die Vorfüllflüssigkeit 27, zu der Doppelport-Kupplungsbuchse 70 gepumpt wird, die von dort zu dem Abfallflüssigkeitsbehälter 28 abfließt.

Während aller Phasen kann über einen geräteinternen Drucksensor 34 im Zulauf zu der Doppelport-Kupplungsbuchse 70 geprüft werden, ob die Doppelportkupplung 15 fluiddicht verkuppelt ist. Dies kann ggf. auch mit weiteren Drucksensoren am Externfluidik-Set 14 erfolgen.
- 10: Hämodialyse-Geräteanordnung
- 12: Basisgerät
- 13: Dialyseflüssigkeits-Versorgung
- 14: Externfluidik-Set
- 15: Koaxial-Doppelportkupplung
- 16: Doppelport-Adapter
- 16': Adapterkörper
- 18: Blutkreis-Leitungsanordnung
- 20: Gerätegehäuse
- 22: Gerätegehäuse-Interface

- 24: Substituatflüssigkeits-Quelle
- 25: Substituatflüssigkeit
- 26: Vorfüllflüssigkeits-Quelle
- 27: Vorfüllflüssigkeit

- 28: Abfallflüssigkeitsbehälter
- 29: Abfallflüssigkeit

- 31: Substituatpumpe
- 32: Vorfüllflüssigkeitspumpe

- 34: Drucksensor

- 36: Quellenwahl-Ventil
- 37: Synchronfüllungs-Ventil

- 40: Verriegelungsmittel
- 41: Verriegelungsteil
- 42: Flussumkehr-Kappe
- 44: Positionsdetektor
- 46: Verriegelungsmittel-Welle
- 47: Vorspannfeder

- 50: Blutpumpe
- 50': Blutpumpen-Mechanik
- 52: Blutpumpen-Antriebsmotor

- 60: Dialysator
- 62: Blutleitung
- 62',62", 62‴: Leitungsabschnitt
- 63: Substituatzugang
- 64: Substituatzugang
- 65: Substituatzugang
- 66: Rückschlagventil
- 67: Luftfalle

- 68: arterielles Leitungsende
- 69: venöses Leitungsende

- 70: Doppelport-Kupplungsbuchse
- 72: Bajonettverriegelung
- 72', 72": Verriegelungssteg, Verriegelungsnase
- 74: Buchsen-Ringleitung
- 76: Buchsen-Zentralleitung
- 77: Dichtring
- 78: Buchsenwand
- 79: Dichtring-Nut

- 80: Doppelport-Kupplungsstecker
- 81: Stecker-Anschlussleitung
- 84: Stecker-Ringleitung
- 86: Stecker-Zentralleitung

- 100: Patientenkörper
- 102: arterieller Patientenzugang
- 104: venöser Patientenzugang

- 110: arterieller Kupplungsstecker
- 111: arterieller Kupplungsstecker

- 120: arterielle Kupplungsbuchse
- 121: arterielle Kupplungsbuchse
- 122: arterielle Kupplungsbuchse
- 123: arterielle Kupplungsbuchse

- 130: venöser Kupplungsstecker
- 131: venöser Kupplungsstecker

- 140: venöse Kupplungsbuchse

## Patentansprüche

1. Hämodialyse-Geräteanordnung (10) mit einem Basisgerät (12), das ein Gerätegehäuse (20) und eine Internfluidik innerhalb des Gerätegehäuses (20) aufweist, und mit einem eine Externfluidik bildenden Externfluidik-Set (14) außerhalb des Gerätegehäuses (20), das die den extrakorporalen Blutkreislauf bildenden Blutleitungen (62) aufweist und die über kuppelbare Fluidkupplungen fluidisch mit dem Basisgerät (12) verbunden ist, wobei eine fluidische Koaxial-Doppelport-Kupplung (15) vorgesehen ist, die von einer Basisgerät-seitigen stationären Doppelport-Buchse (70) und einem korrespondierenden Externfluidik-Set-seitigen Doppelport-Stecker (80) gebildet ist, der auf die Doppelport-Buchse (70) lösbar aufgesteckt ist,
wobei sowohl der Doppelport-Stecker (80) als auch die Doppelport-Buchse (70) jeweils eine fluidische Zentralleitung (86,76) und eine die Zentralleitung (86,76) jeweils ringförmig umgebende koaxiale fluidische Ringleitung (84,74) aufweisen,
wobei dem Basisgerät (12) fluidisch eine Substituatflüssigkeits-Quelle (24) zugeordnet ist, die fluidisch mit der Buchsen-Ringleitung (74) oder der Buchsen-Zentralleitung verbunden ist, und
wobei dem Basisgerät (12) ein Abfallflüssigkeits-Ziel (28) zugeordnet ist, das fluidisch mit der Buchsen-Zentralleitung (76) oder der Buchsen-Ringleitung verbunden ist.

2. Hämodialyse-Geräteanordnung (10) nach Anspruch 1, wobei geräteseitig ein Verriegelungsmittel (40) vorgesehen ist, wobei das Verriegelungsmittel (40) in seiner Verriegelungsposition den Doppelport-Stecker (80) in seiner Kupplungsposition auf der stationären Doppelport-Buchse (70) blockiert.

3. Hämodialyse-Geräteanordnung (10) nach Anspruch 2, wobei das Verriegelungsmittel (40) eine Flussumkehr-Kappe (42) aufweist, die bei nicht angekoppeltem Doppelport-Stecker (80) in einer Spülposition des Verriegelungsmittels (40) die Doppelport-Buchse (70) derart verschließt, dass die Buchsen-Zentralleitung (76) fluidisch verbunden ist mit der Buchsen-Ringleitung (74).

4. Hämodialyse-Geräteanordnung (10) nach einem der vorangegangenen Ansprüche, wobei dem Verriegelungsmittel (40) ein Positionsdetektor (44) zugeordnet ist, der die Verriegelungsposition und/oder die Spülposition des Verriegelungsmittels (40) detektiert.

5. Hämodialyse-Geräteanordnung (10) nach Anspruch 4, wobei das Basisgerät (12) eine interne Substituatpumpe (31) aufweist, die die Substituatflüssigkeit (25) von der Substituatflüssigkeits-Quelle (24) zu der Doppelport-Kupplung (15) pumpt.

6. Hämodialyse-Geräteanordnung (10) nach einem der vorangegangenen Ansprüche, wobei das Externfluidik-Set (14) einen Doppelport-Adapter (16) mit dem Doppelport-Stecker (80) und eine separate Blutkreis-Leitungsanordnung (18) aufweist, wobei die Blutkreis-Leitungsanordnung (18) an ihrem arteriellen und ihrem venösen Leitungsende (68,69) eine arterielle Kupplungsbuchse (120) und eine venöse Kupplungsbuchse (140) aufweist, und wobei der Doppelport-Adapter (16) einen korrespondierenden arteriellen Kupplungsstecker (110) aufweist, der fluidisch mit der Substituatflüssigkeits-Quelle (24) verbunden ist, und einen korrespondierenden venösen Kupplungsstecker (130) aufweist, der fluidisch mit dem Abfallflüssigkeitsbehälter (28) verbunden ist.

7. Hämodialyse-Geräteanordnung (10) nach einem der vorangegangenen Ansprüche, wobei die Blutkreis-Leitungsanordnung (18) einen separaten Substituatzugang (63,64, 65) zwischen den beiden Blutkreis-Leitungsenden (68,69) aufweist, der von einer arteriellen Kupplungsbuchse (121,122,123) gebildet wird.

8. Hämodialyse-Geräteanordnung (10) nach einem der vorangegangenen Ansprüche, wobei stromaufwärts des venösen Leitungsendes (69) ein Rückschlagventil (66) angeordnet ist.

## Claims

1. Heamodialysis machine assembly (10) comprising a base unit (12) which comprises a machine housing (20) and an internal fluidics within the machine housing (20), and an external fluidic set (14) forming an external fluidics outside the machine housing (20), which comprises the blood lines (62) defining the extracorporeal blood circuit and which is fluidically connected to the base unit (12) via couplable fluid couplings, wherein there is provided a fluidic coaxial dual port coupling (15) defined by a base unit side stationary dual port socket (70) and a corresponding extracorporeal fluidic set side dual port plug (80) releasably plugged onto the dual port socket (70),
wherein both the dual port plug (80) and the dual port socket (70) comprise respectively a fluidic central line (86, 76) and a coaxial fluidic ring line (84, 74) surrounding the central line (86, 76) in an annular manner respectively,
wherein a substitute liquid source (24) is fluidically associated with the base unit (12) and is fluidically connected to the socket ring line (74) or the socket central line, and
wherein the base unit (12) has associated therewith a waste fluid target (28) fluidically connected to the socket central line (76) or the socket ring line.

2. Haemodialysis machine assembly (10) according to claim 1, wherein a locking means (40) is provided at the machine side, wherein the locking means (40) in its locking position locks the dual port plug (80) in its coupling position at the stationary dual port socket (70).

3. Haemodialysis machine assembly (10) according to claim 2, wherein said locking means (40) comprises a flow reversal cap (42) which, when said dual port plug (80) is not coupled in a flushing position of said locking means (40), closes said dual port socket (70) in such a way that said socket central line (76) is fluidically connected to said socket ring line (74).

4. Haemodialysis machine assembly (10) according to any one of the preceding claims, wherein the locking means (40) is provided with a position detector (44) which detects the locking position and/or the flushing position of the locking means (40).

5. Haemodialysis machine assembly (10) according to claim 4, wherein said base unit (12) comprises an internal substituent pump (31) pumping said substituent fluid (25) from said substitute liquid source (24) to said dual port coupling (15).

6. Haemodialysis machine assembly (10) according to any one of the preceding claims, wherein the external fluidic set (14) comprises a dual port adapter (16) with the dual port plug (80) and a separate blood circuit line assembly (18), wherein the blood circuit line assembly (18) comprises at its arterial and venous conduit ends (68, 69) an arterial coupling socket (120) and a venous coupling socket (140), and wherein the dual port adapter (16) comprises a corresponding arterial coupling plug (110) fluidically connected to the substitute liquid source (24) and a corresponding venous coupling plug (130) fluidically connected to the waste liquid container (28).

7. Haemodialysis machine assembly (10) according to any one of the preceding claims, wherein the blood circuit line assembly (18) comprises a separate substitute access (63,64,65) between the two blood circuit line ends (68,69) defined by an arterial coupling receptacle (121,122,123).

8. Haemodialysis machine assembly (10) according to any one of the preceding claims, wherein a check valve (66) is arranged upstream of the venous line end (69).

## Revendications

1. Ensemble d'appareil d'hémodialyse (10) avec un unité de base (12) qui comprend un boîtier d'unité (20) et un système fluidique interne à l'intérieur du boîtier d'unité (20), et avec un set extra-fluidique (14) formant un système fluidique externe à l'extérieur du boîtier d'unité (20), qui comprend les conduites de sang (62) formant le circuit de sang extracorporel et qui est relié fluidiquement à l'unité de base (12) par des connexions fluidiques pouvant être couplées,
dans lequel il est prévu un couplage coaxial fluidique à double port (15) qui est formé par une douille stationnaire à double port (70) côté appareil de base et une fiche à double port (80) correspondante côté set extra-fluidique, qui est enfichée de manière détachable sur la douille à double port (70),
la fiche à double port (80) et la douille à double port (70) comprenant chacune une conduite centrale fluidique (86, 76) et une conduite annulaire fluidique coaxiale (84, 74) entourant la conduite centrale (86, 76) de manière annulaire,
dans lequel une source de liquide de substitution (24) est associée de manière fluidique à l'unité de base (12), laquelle est reliée de manière fluidique à la conduite annulaire à douilles (74) ou à la conduite centrale à douilles, et
dans lequel une cible de liquide de déchets (28) est associée à l'unité de base (12) et est connectée de manière fluidique à la conduite centrale à douilles (76) ou à la conduite annulaire à douilles.

2. Ensemble d'appareils d'hémodialyse (10) selon la revendication 1, dans lequel un moyen de verrouillage (40) est prévu du côté de l'unite, le moyen de verrouillage (40) bloquant, dans sa position de verrouillage, le connecteur à double port (80) dans sa position d'accouplement sur le connecteur douillé stationnaire à double port (70).

3. Ensemble d'appareils d'hémodialyse (10) selon la revendication 2, dans lequel le moyen de verrouillage (40) comprend un capuchon d'inversion de flux (42) qui, lorsque le connecteur double port (80) n'est pas couplé dans une position de rinçage du moyen de verrouillage (40), obture le connecteur double port (70) de telle sorte que le conduit central de connecteur (76) est en communication fluidique avec le conduit annulaire de connecteur (74).

4. Ensemble d'appareils d'hémodialyse (10) selon l'une quelconque des revendications précédentes, dans lequel le moyen de verrouillage (40) est associé à un détecteur de position (44) qui détecte la position de verrouillage et/ou la position de rinçage du moyen de verrouillage (40).

5. Ensemble d'appareils d'hémodialyse (10) selon la revendication 4, dans lequel l'unité de base (12) comprend une pompe de substitution interne (31) qui pompe le liquide de substitution (25) de la source de liquide de substitution (24) vers le couplage à double port (15).

6. Ensemble d'appareils d'hémodialyse (10) selon l'une quelconque des revendications précédentes, dans lequel le set extra-fluidique (14) comprend un adaptateur à double port (16) avec le connecteur à double port (80) et un ensemble de conduites de circuit sanguin séparé (18), dans lequel le set de conduites de circuit sanguin (18) comprend à ses terminaisons de conduite artérielle et veineuse (68, 69) comprend un raccord artériel femelle (120) et un raccord veineux femelle (140), et dans lequel l'adaptateur à double port (16) comprend un raccord artériel mâle correspondant (110) qui est connecté de manière fluidique à la source de liquide de substitution (24) et un raccord veineux mâle correspondant (130) qui est connecté de manière fluidique au réservoir de liquide de déchets (28).

7. Ensemble d'appareils d'hémodialyse (10) selon l'une quelconque des revendications précédentes, dans lequel l'ensemble de conduits de circuit sanguin (18) comprend un accès de substitution séparé (63, 64, 65) entre les deux terminaisons de conduits de circuit sanguin (68, 69), formé par une douille de couplage artériel (121, 122, 123).

8. Ensemble d'appareils d'hémodialyse (10) selon l'une quelconque des revendications précédentes, dans lequel un clapet anti-retour (66) est disposé en amont de la terminaison de ligne veineuse (69).
